# FASCICULE DE BREVET EUROPEEN

(11) **EP 1 309 357 B1**
(45) Date de publication et mention de la délivrance du brevet: **18.11.2009**
(21) Numéro de dépôt: 01963074.8
(22) Date de dépôt: 08.08.2001
(51) Int. Cl.: A61L 2/04

(54) **PROCEDE ET DISPOSITIF DE TRAITEMENT SANITAIRE SIMULTANE D'UN FLUIDE ET DE SON RESEAU**
VERFAHREN UND VORRICHTUNG ZUR GLEICHZEITIGEN SANITÄREN BEHANDLUNG EINER FLÜSSIGKEIT UND IHRES LEITUNGSSYSTEMS
METHOD AND DEVICE FOR SIMULTANEOUS SANITARY TREATMENT OF A FLUID AND ITS DISTRIBUTION NETWORK

(30) Priorité: 08.08.2000 FR 0010442
(43) Date de publication de la demande: 14.05.2003
(73) Titulaire: Boiffier, Jean-Jacques, 17100 Saintes (FR)
(72) Inventeur: BOIFFIER, Jean-Jacques, F-17100 Saintes (FR)
(74) Mandataire: Maupilier, Didier
(86) Numéro de dépôt international: PCT/FR2001/002573
(87) Numéro de publication internationale: WO 2002/011773

(56) Documents cités:
- EP-A- 0 052 926
- EP-A- 0 705 608
- FR-A- 2 652 005
- US-A- 5 603 894

## Description

La présente invention concerne un procédé et un dispositif de traitement sanitaire simultané d'un fluide et de son réseau, notamment dans un milieu sensible comme le milieu hospitalier et plus généralement un lieu recevant du public.

Dans le secteur hospitalier, on sait que certains micro-organismes se développent comme dans beaucoup d'autres réseaux mais la spécificité de ce type d'établissement est particulier pour au moins deux raisons.

La première est que la densité de la faune microbienne est plus élevée qu'ailleurs et que cette faune est souvent partiellement insensibilisée à certains traitements.

La seconde est que les personnes qui fréquentent ces établissements présentent souvent un déficit immunitaire si bien qu'elles sont plus sensibles à l'agression de tels micro-organismes.

Un vecteur particulièrement sensible qui permet le développement et la diffusion de tels micro-organismes est l'eau chaude sanitaire.

En effet, on trouve là une température suffisamment élevée pour assurer le développement de cette faune mais insuffisante pour la tuer par action thermique car l'eau chaude sanitaire doit être distribuée à une température compatible avec la résistance de l'épiderme et du derme même en cas d'erreur de manipulation du distributeur d'eau.

De plus, les parties humides permettent aussi de créer une ambiance spécifiquement propice à un développement important et rapide.

C'est ainsi que des maladies comme la légionellose peuvent se développer avec des conséquences irrémédiables. Le germe responsable de cette maladie est un bacille vivant dans l'eau douce dont la température optimale de prolifération se situe entre 35 et 40 °C. L'infection est provoquée lors d'une inhalation d'un aérosol d'eau contaminée comme cela est le cas lors d'un passage à la douche.

Aussi, une préoccupation des milieux de soins et des établissements sensibles en général est de lutter contre de tels développements de micro-organismes.

On sait aussi que dans le cas d'eaux stagnantes et dans le cas de faibles températures de circulation, il se produit un biofilm qui est un dépôt de particules composé d'oxydes de fer et de sédiments, dans lequel les bactéries se développent particulièrement bien et dans lequel elles se trouvent protégées des agressions constituées par des pics de température par exemple sauf à aller largement au-dessus des seuils et pendant une durée adaptée.

Ainsi pour les légionelles, à 50 °C, elles sont simplement inertées mais se développent dès que la température diminue. Par contre, à 60 °C il faut 32 minutes pour provoquer la destruction de ces micro-organismes et à 75 °C, une minute suffit pour les éradiquer.

Dans l'industrie, par exemple l'industrie alimentaire, il faut aussi pouvoir traiter les circuits de transfert et d'élaboration de produits alimentaires en vue de limiter la prolifération d'organismes pathogènes.

Si ce problème de pollution bactérienne se pose pour les adductions de liquides, il en va de même pour les effluents.

Pour le cas simple de l'eau, on connaît des traitements par ajout de désinfectants dans l'eau distribuée mais on comprend que les doses ne peuvent être que minimes pour que cette eau reste potable et de plus, le pouvoir désinfectant de certains composés diminue par une accoutumance des micro-organismes à de telles substances.

En outre, on sait que certains micro-organismes sont particulièrement résistants à ces désinfectants.

Un moyen sûr de désinfecter un élément liquide ou solide reste la stérilisation à chaud. Si cela est relativement simple pour des outils chirurgicaux ou pour du linge ou encore pour des ustensiles, cela reste beaucoup plus complexe pour un fluide distribué ou un effluent.

A cet effet, selon l'invention, le procédé de traitement sanitaire simultané d'un fluide et de son réseau, notamment pour l'éradication des micro-organismes pathogènes, se **caractérise en ce qu**'il consiste en la succession des étapes suivantes :
- alimentation en fluide à traiter,
- élévation de ce fluide à une température T_{P} de pasteurisation pendant une durée t_{P} de pasteurisation fonction de cette température T_{P},
- abaissement de la température du fluide à une valeur T_{D} < T_{P} dite valeur de distribution mais supérieure à la température d'éradication des micro-organismes,
- circulation en continu de ce fluide à la température T_{D} en continu à travers le réseau vers les postes de distribution en sorte de détruire le biofilm constitué et d'interdire la constitution d'un nouveau biofilm, et
- rebouclage d'une partie au moins du fluide non consommé pour le maintenir à cette température T_{D.}

Selon une autre caractéristique, on réalise préalablement à toute mise en service, une étape de circulation à travers l'ensemble du réseau du fluide porté à la température T_{P} pendant une durée supérieure à la durée d'éradication.

On peut aussi procéder à une première élévation de température du fluide entrant par échange avec le fluide sortant porté à la température T_{P.}

Le dispositif comprend plus spécifiquement ainsi que cela va être détaillé par la suite :
- une alimentation en fluide à traiter à une température d'entrée T_{E},
- un circuit de pasteurisation de ce fluide à une température T_{P} pendant une durée t_{P},
- un échangeur prévu pour abaisser la température du fluide à une valeur T_{D} < T_{P} dite valeur de distribution mais supérieure à la température d'éradication des micro-organismes,
- un circuit de distribution de ce fluide à la température I_{D} vers les postes de distribution, et
- un circuit de rebouclage d'une partie au moins du fluide non consommé pour le maintenir à cette température T_{D}.

De façon préférentielle, on utilise un échangeur double flux qui augmente la température de ce fluide entrant d'une valeur ΔT donnée et qui diminue la température T_{P} du fluide pasteurisé de cette même valeur ΔT.

Pour les besoins de la description d'un mode préférentiel de réalisation, pour simplifier les éléments nécessaires et parce que l'application aux réseaux de distribution d'eau chaude sanitaire est un problème important, ceci pour de nombreux lieux et plus particulièrement pour ceux qui reçoivent du public, c'est ce cas d'application qui est retenu.

L'avantage de la présente invention est de permettre le maintien du fluide en circulation permanente dans le circuit de distribution sous traitement, tout en assurant le traitement simultané, en continu du réseau. Le traitement est un traitement à la chaleur par pasteurisation ainsi que cela est expliqué ci-après, en regard des dessins annexés sur lesquels les figures représentent un mode de réalisation préférentiel, non limitatif:
- figure 1, une vue d'un circuit général montrant le fonctionnement du dispositif de traitement selon la présente invention, et
- figure 2, une vue d'un circuit complet eau chaude / eau froide à débit contrôlé et des postes d'utilisation.

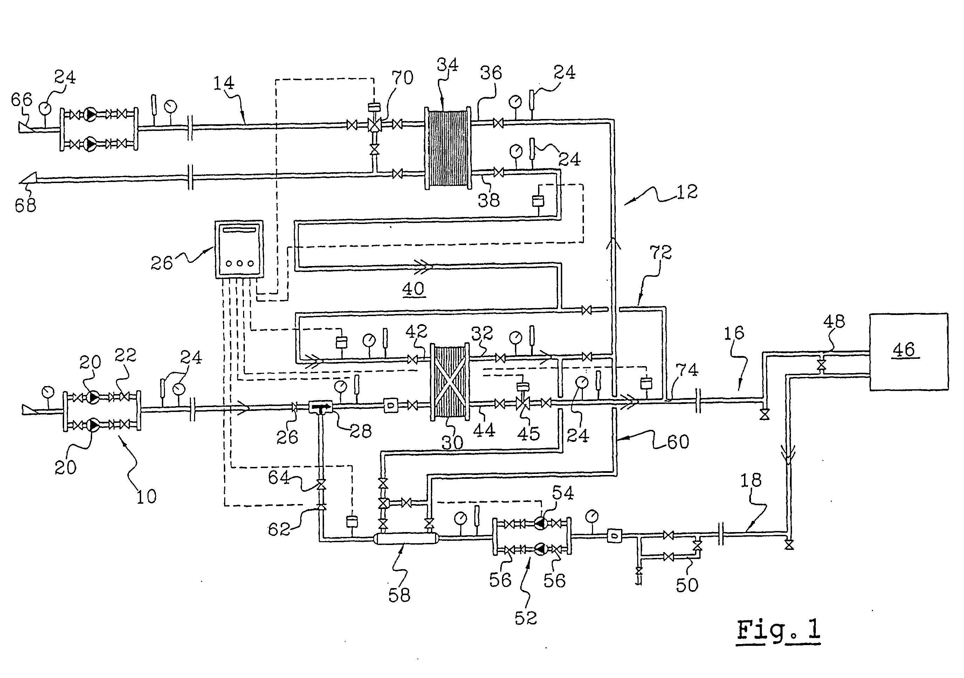

Sur la figure 1, on a représenté en 10 l'alimentation en eau froide à partir du réseau public, en 12 le circuit de pasteurisation, en 14 le circuit primaire et en 16 le circuit de distribution d'eau chaude sanitaire avec son circuit de rebouclage 18.

Le circuit d'alimentation en eau froide est généralement sous pression de distribution 3 à 4 bars et une pression complémentaire est éventuellement ajoutée au moyen de pompes 20 équipées de vannes 22 de sectionnement adaptées.

De façon générale, l'instrumentation est référencée 24 et elle comprend des manomètres, des capteurs de température, des piquages de prélèvement, des soupapes de sécurité et tout autre appareillage rendu nécessaire sur un circuit de distribution sous pression. Cet équipement connexe est connu de l'homme de l'art et n'intéresse pas directement l'invention.

Cette instrumentation est pilotée par un automate programmable qui assure la conduite des organes mobiles du circuit en fonction des mesures effectuées et des valeurs de consigne comme cela sera expliqué ultérieurement.

Ce circuit d'alimentation en eau froide comporte un clapet 26 anti-retour précédant un Té 28 de raccordement ou mélangeur par effet Venturi donc à addition de pressions. Ce Té est raccordé à un échangeur 30 double flux. Ce circuit débouche ensuite sur le circuit 12 de pasteurisation après passage dans cet échangeur dont on verra les particularités et les actions lors de la description du fonctionnement.

L'eau d'alimentation, après passage dans l'échangeur 30 double flux, sort en 32 pour aller dans l'échangeur 34 de pasteurisation dans lequel elle entre en 36 et duquel elle sort en 38.

Cette eau circule alors dans une lyre 40 de pasteurisation qui comprend une longueur importante de tuyauterie. Pour donner un exemple, un débit de 10m³/h nécessite de l'ordre de 30m de tuyauterie d'un diamètre nominal 100 pour engendrer une durée de circulation adaptée.

Cette lyre, en sortie, pénètre de nouveau dans l'échangeur 30 double flux en 42 pour en sortir en 44 à travers une électrovanne 45 dans le circuit 16 de distribution d'eau chaude sanitaire, l'ensemble des postes d'eau chaude sanitaire étant symbolisé en 46.

Ce circuit 16 de distribution comporte le circuit 18 de rebouclage qui est piqué en 48, au plus près de chacun des postes de soutirage en sorte d'éviter toute zone morte. Ce circuit de rebouclage traite sensiblement 20 à 30% de l'eau du réseau complet.

Sur ce circuit de rebouclage, il est prévu une boucle 50 de prélèvement permettant la prise d'échantillons aux fins d'analyses, un ensemble 52 de mise en circulation du fluide dans cette boucle par un groupe de pompes 54 et de vannes 56 de sectionnement, et un échangeur 58 simple monté en dérivation par un circuit 60 aller/retour sur le circuit de pasteurisation.

Ce circuit est complété par un clapet 62 anti-retour et une vanne 64 d'isolement, en amont du Té 28 de raccordement ou mélangeur.

Le circuit 14 primaire comprend une alimentation 66 en fluide caloporteur avec un retour en 68 après passage et échange thermique dans l'échangeur 34 de pasteurisation.

Une électrovanne 70 permet de régler le débit en fonction des mesures recueillies par l'instrumentation 24.

Ce circuit primaire, tuyauterie et échangeur, est souvent la solution retenue mais cet ensemble peut être remplacé par une source de chaleur directe telle qu'une chaudière, le but étant dans les deux cas d'apporter les calories nécessaires pour une pasteurisation du fluide circulant. Le choix résulte des disponibilités in situ comme par exemple la présence d'une centrale importante de chauffage centralisé susceptible d'assurer aussi le chauffage de l'eau sanitaire et sa pasteurisation.

La présente invention propose également un agencement d'une dérivation 72 curative, dite aussi by-pass thermique, qui est piquée sur la lyre 40 de pasteurisation et qui débouche directement dans le circuit 16 de distribution d'eau chaude en un point 74, en amont des postes 46 d'eau chaude sanitaire.

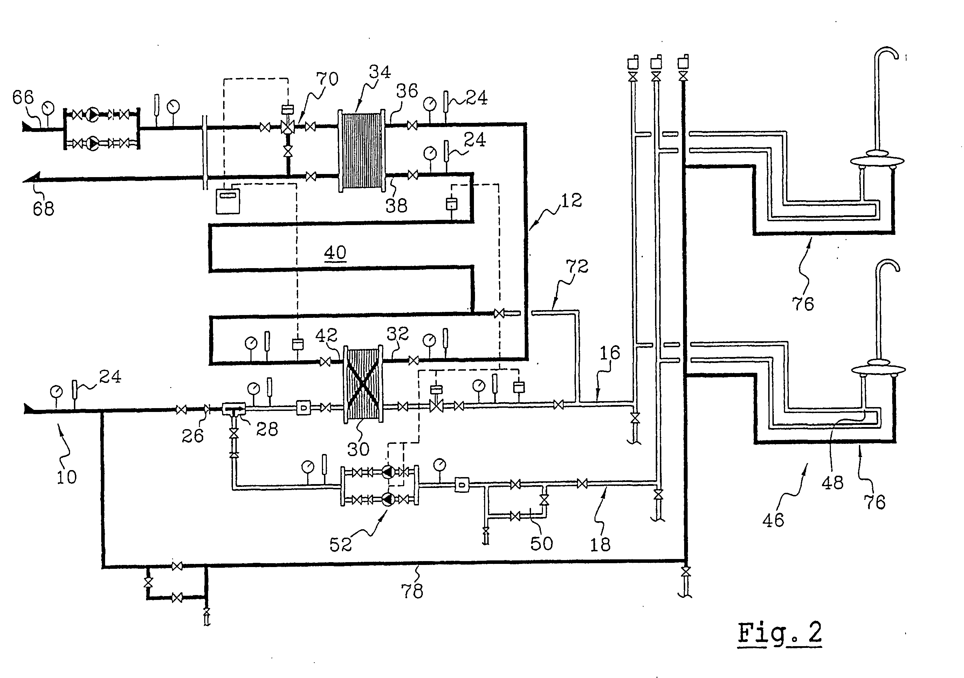

Sur la figure 2, on a repris les mêmes éléments avec les mêmes références en ajoutant un exemple schématique de deux postes 76 de distribution et le circuit 78 d'eau froide.

Sur ce schéma fluide, pour sa clarté, certains éléments complémentaires ont été supprimés comme l'échangeur 58 simple. En effet, dans certaines applications, cet échangeur simple ne présente pas d'intérêt. Cet échangeur est surtout prévu lorsque le fluide est rebouclé mais qu'il a subi dans l'intervalle une forte modification de sa température à la hausse comme à la baisse. Dans ce cas, l'échangeur permet de ramener le fluide à la température adéquate en amont du Té de raccordement ou de mélange.

Le fonctionnement d'une telle installation et du dispositif selon la présente invention est maintenant décrit en regard des deux figures.

L'eau froide est tirée au niveau de chacun des postes 76 de distribution à la pression du réseau. L'eau, traitée au préalable et restant à la température ambiante, de l'ordre de 10 à 15 °C, ne permet pas la prolifération des micro-organismes comme l'eau chaude sanitaire à 40°C dont on sait qu'elle favorise les développements de micro-organismes.

L'eau froide destinée à fabriquer de l'eau chaude sanitaire, arrive à température ambiante d'entrée T_{E} de l'ordre de 10 à 15°C, passe à travers l'ensemble de pompage 20/22 et à travers le Té 28, elle entre en contact avec l'eau chaude du circuit de rebouclage qui est à une température de 60°C, dite de distribution T_{D} , à quelques degrés dus aux pertes en ligne.

Cette eau mélangée est sensiblement à une température T_{E} de 20 à 25°C après mélange en consommation maximale et à 60°C s'il n'y a eu aucun soutirage. Cette eau passe alors à travers l'échangeur 30 double flux. Dans cet échangeur, elle gagne un ΔT prédéterminé, dans notre exemple préférentiel ce ΔT est fixé à 15°C au flux sortant comme cela va être expliqué.

Ce mélange ayant subi un gain de calories passe ensuite à travers l'échangeur 34 de pasteurisation dans lequel il subit une élévation de température complémentaire jusqu'à la température T_{P} de pasteurisation, en l'occurrence 75°C environ.

La lyre 40 de pasteurisation permet de maintenir l'eau ainsi chauffée à cette température T_{P} pendant une durée t_{P} suffisante qui assure la destruction des micro-organismes. II est préférable de prévoir un module spécifique, qui est d'une grande compacité en sorte d'éviter les pertes calorifiques et de limiter l'encombrement.

Par contre, cette eau chaude sortant de la lyre est à une température T_{P} supérieure à la température T_{D} admissible de distribution.

C'est à travers l'échangeur 30 double flux que cette eau perd le même ΔT de 15°C. II y a donc une optimisation le bilan doit être nul dans cet échangeur , avec les économies d'énergie correspondantes.

On comprend que, dans le bilan, les calories sont apportées uniquement par le circuit primaire.

Cette eau à température admissible peut alors être distribuée et utilisée, sans qu'elle comporte d'éléments pathogènes.

Cette eau ne stagne aucunement dans les canalisations car elle est en perpétuelle circulation grâce au circuit 18 de rebouclage. En effet, soit le débit d'eau pasteurisée produit est intégralement consommé, soit il est consommé en partie seulement et une partie passe dans le circuit de rebouclage, soit il n'est pas du tout consommé et circule intégralement dans le circuit de rebouclage mais il n'y a aucune stagnation.

Ainsi l'eau circule à 60°C et il y a en permanence une double pasteurisation du fluide d'une part et des canalisations d'autre part. En effet, le biofilm ne peut pas se former et le biofilm existant est même détruit.

Selon une autre possibilité de l'invention, dans une installation existante avec un biofilm constitué ou lorsque le circuit est interrompu pendant une longue durée ou encore lors d'une première mise en service, on procède à un traitement curatif préalable. Ce traitement consiste à faire circuler pendant une durée déterminée de l'eau à la température de pasteurisation T_{P} de 75°C dans l'ensemble du circuit. Ceci permet d'éprouver l'installation complète et la résistance de ses composants tout en traitant toutes les parties de cette installation. Pour ce faire, la dérivation 72 permet d'introduire directement cette eau à partir de la lyre, c'est-à-dire à 75°C dans le cas préférentiel retenu.

On constate aussi que l'eau froide entrante subit un premier mélange au contact de l'eau chaude de rebouclage, avant même son passage dans l'échangeur double flux. Ceci est une action qui se produit uniquement s'il y a eu soutirage sinon, l'eau chaude traitée par pasteurisation fait de nouveau le cycle complet à travers le circuit, supportant à nouveau une pasteurisation à 75°C.

Les procédé et dispositif selon la présente invention permettent de traiter ainsi simultanément le fluide et son réseau, ce qui est particulièrement attractif.

Le mode de réalisation traite d'une application à l'eau chaude sanitaire mais il est possible d'appliquer le procédé comme le dispositif aux effluents avant rejet dans le réseau public d'assainissement ou aux produits fluides tels que le lait dans l'agroalimentaire.

## Revendications

1. Procédé de traitement sanitaire simultané d'un fluide et de son réseau pour l'éradication des micro-organismes pathogènes, **caractérisé en ce qu'**il consiste en la succession des étapes suivantes :
- alimentation en fluide à traiter,
- élévation de ce fluide à une température T_{P} de pasteurisation pendant une durée t_{P} de pasteurisation,
- abaissement de la température du fluide à une valeur T_{D} < T_{P} dite valeur de distribution mais supérieure à la température d'éradication des micro-organismes,
- circulation en continu de ce fluide à la température T_{D} à travers le réseau vers les postes de distribution en sorte de détruire le biofilm constitué et d'interdire la constitution d'un nouveau biofilm, et
- rebouclage sans stagnation du fluide non consommé pour le maintenir à cette température T_{D}.

2. Procédé de traitement sanitaire selon la revendication 1, **caractérisé en ce que**, dans le cas d'une installation existante, d'une installation ayant subi un arrêt prolongé, d'une installation nouvelle, on réalise préalablement à toute mise en service une étape de circulation, à travers l'ensemble du réseau, du fluide porté à la température T_{P} pendant une durée supérieure à la durée d'éradication.

3. Procédé de traitement sanitaire selon la revendication 1 ou 2, **caractérisé en ce que**, préalablement à l'élévation de ce fluide à une température T_{P} de pasteurisation pendant une durée t_{P} de pasteurisation fonction de cette température T_{P}, on procède à une première élévation de température du fluide entrant par échange avec le fluide sortant porté à la température T_{P}.

4. Procédé de traitement sanitaire selon l'une des revendications 1 à 3, **caractérisé en ce que** les différentes valeurs des températures sont:
- T_{P}= 75°C,
- T_{D}= 60°C

5. Dispositif de traitement sanitaire permettant la mise en oeuvre du procédé selon la revendication 1 à 4, **caractérisé en ce qu'**il comprend :
- une alimentation (10) en fluide à traiter à une température d'entrée T_{E} reliée à un Té de raccordement ou mélangeur par effet Venturi,
- un circuit (12) de pasteurisation de ce fluide à une température T_{P} pendant une durée t_{P},
- un échangeur (30) pour abaisser la température du fluide à une valeur T_{D} < T_{P} dite valeur de distribution mais supérieure à la température d'éradication des micro-organismes,
- un circuit (16) de distribution de ce fluide à la température T_{D} vers les postes (46,76) de distribution, et
- un circuit (18) de rebouclage sans stagnation du fluide non consommé pour le maintenir à cette température T_{D}, ledit circuit de rebouclage étant raccordé audit Té de raccordement ou mélangeur par effet Venturi.

6. Dispositif de traitement sanitaire selon la revendication 5, **caractérisé en ce que** l'échangeur (30) est un échangeur double flux qui augmente la température de ce fluide entrant d'une valeur ΔT donnée pour atteindre une valeur T_{E}+ΔT et qui diminue la température T_{P} du fluide pasteurisé de cette même valeur pour atteindre une température de distribution T_{D} = T_{P}-ΔT.

7. Dispositif de traitement sanitaire selon la revendication 5 ou 6, **caractérisé en ce que** le circuit (12) de pasteurisation comprend un circuit primaire (14) avec un échangeur (34) de pasteurisation.

8. Dispositif de traitement sanitaire selon l'une quelconque des revendications précédentes 5, 6 ou 7, **caractérisé en ce que** le circuit (12) de pasteurisation comprend une lyre (40) de pasteurisation.

9. Dispositif de traitement sanitaire selon l'une quelconque des revendications 5 à 8, **caractérisé en ce qu'**il comprend une dérivation (72) curative ou by-pass thermique interposée entre le circuit (12) de pasteurisation et le circuit (16) de distribution.

10. Dispositif de traitement sanitaire selon l'une quelconque des revendications 5 à 9, **caractérisé en ce qu'**il comprend un échangeur (58) simple monté sur le circuit (18) de rebouclage.

## Claims

1. A method for the simultaneous sanitary treatment of a fluid and its system for eradicating pathogenic microorganisms, **characterised in that** it comprises a succession of the following steps:
- supplying fluid to be treated,
- raising this fluid to a pasteurisation temperature T_{P} for a pasteurisation period tₚ,
- lowering the temperature of the fluid to a value T_{D} < Tₚ referred to as the distribution value but higher than the micro-organism eradication temperature,
- continuously circulating this fluid at the temperature T_{D} through the system to the distribution station so as to destroy the biofilm formed and to prevent the formation of a new biofilm, and
- looping back the unconsumed fluid without stagnation to maintain it as this temperature T_{D}.

2. A sanitary treatment method according to claim 1, **characterised in that**, in the case of an existing installation, an installation that has undergone prolonged stoppage, or a new installation, a step of circulating fluid raised to the temperature Tₚ is carried out, before any commissioning, through the whole of the system for a period longer than the eradication period.

3. A sanitary treatment method according to claim 1 or 2, **characterised in that**, prior to the raising of this fluid to a pasteurisation temperature Tₚ for a pasteurisation period tₚ that depends on this temperature Tₚ, a first raising of temperature of the incoming fluid is carried out by exchange with the outgoing fluid raised to the temperature Tₚ.

4. A sanitary treatment method according to one of claims 1 to 3, **characterised in that** the different values of the temperatures are:
- Tₚ = 75°C
- T_{D} = 60°C

5. A sanitary treatment device for implementing the method according to claims 1 to 4, **characterised in that** it comprises:
- a supply (10) of fluid to be treated at an incoming temperature Tₑ connected to a connecting Tee or mixer by venturi effect,
- a circuit (12) for pasteurising this fluid at a temperature Tₚ for a period tₚ,
- an exchanger (30) for reducing the temperature of the fluid to a value T_{D} < Tₚ referred to as the distribution value but higher than the micro-organism eradication temperature,
- a circuit (16) for distributing this fluid at the temperature T_{D} to the distribution stations (46, 76), and
- a circuit (18) for looping back without stagnation of the unconsumed fluid in order to maintain it at this temperature T_{D}, said looping-back circuit being connected to said connection Tee or mixer by venturi effect.

6. A sanitary treatment device according to claim 5, **characterised in that** the exchanger (30) is a double-flow exchanger that increases the temperature of this incoming fluid by a given value ΔT in order to reach a value T_{E} + ΔT and reduces the temperature Tₚ of the pasteurised fluid by the same value in order to reach a distribution temperature T_{D} = Tₚ - ΔT.

7. A sanitary treatment device according to claim 5 or 6, **characterised in that** the pasteurisation circuit (12) comprises a primary circuit (14) with a pasteurisation exchanger (34).

8. A sanitary treatment device according to any one of the preceding claims 5, 6 or 7, **characterised in that** the pasteurisation circuit (12) comprises a pasteurisation loop (40).

9. A sanitary treatment device according to any one of claims 5 to 8, **characterised in that** it comprises a curative diversion (72) or thermal bypass interposed between the pasteurisation circuit (12) and the distribution circuit (16).

10. A sanitary treatment device according to any one of claims 5 to 9, **characterised in that** it comprises a simple exchanger (58) mounted on the looping-back circuit (18).

## Patentansprüche

1. Verfahren zur gleichzeitigen sanitären Behandlung eines Fluids und seines Verteilungsnetzes zur Ausrottung pathogener Mikroorganismen, **dadurch gekennzeichnet, dass** es nacheinander aus den folgenden Schritten besteht:
- Zuführen eines zu behandelnden Fluids,
- Erhöhen der Temperatur dieses Fluids auf eine Pasteurisierungstemperatur T_{P} während einer Pasteurisierungsdauer t_{P},
- Absenken der Temperatur des Fluids auf einen Wert T_{D} < T_{P}, der Verteilungswert genannt wird, jedoch höher ist als die Temperatur zur Ausrottung der Mikroorganismen,
- kontinuierliches Umwälzen dieses Fluids mit der Temperatur T_{D} durch das Verteilungsnetz zu den Verteilungsstationen, derart, dass der gebildete Biofilm zerstört wird und die Bildung eines neuen Biofilms verhindert wird, und
- Wiedereinleiten ohne Stillstand des nicht verbrauchten Fluids, um es auf dieser Temperatur T_{D} zu halten.

2. Sanitärbehandlungsverfahren nach Anspruch 1, **dadurch gekennzeichnet, dass** im Fall einer bestehenden Installation, einer Installation, die für längere Zeit außer Betrieb war, und einer neuen Installation vor jeder Inbetriebnahme ein Schritt des Umwälzens des auf der Temperatur T_{P} gehaltenen Fluids durch das gesamte Verteilungsnetz während einer Dauer, die länger als die Ausrottungsdauer ist, ausgeführt wird.

3. Sanitärbehandlungsverfahren nach Anspruch 1 oder 2, **dadurch gekennzeichnet, dass** vor der Erhöhung der Temperatur dieses Fluids auf eine Pasteurisierungstemperatur T_{P} während einer Pasteurisierungsdauer t_{P}, die von dieser Temperatur T_{P} abhängt, eine erste Erhöhung der Temperatur des eintretenden Fluids durch Austausch mit dem auf der Temperatur T_{P} gehaltenen austretenden Fluid vorgenommen wird.

4. Sanitärbehandlungsverfahren nach einem der Ansprüche 1 bis 3, **dadurch gekennzeichnet, dass** die verschiedenen Temperaturwerte sind:
- T_{P} = 75 °C,
- T_{D} = 60°C.

5. Sanitärbehandlungsvorrichtung, die das Verfahren nach Anspruch 1 bis 4 ausführt, **dadurch gekennzeichnet, dass** sie umfasst:
- eine Versorgung (10) für zu behandelndes Fluid mit einer Eintrittstemperatur T_{E}, die mit einem Verbindungs-T-Stück oder mit einem Mischer durch Venturi-Wirkung verbunden ist,
- einen Kreis (12) zur Pasteurisierung dieses Fluids auf einer Temperatur T_{P} während einer Dauer t_{P},
- einen Tauscher (30) zur Absenkung der Temperatur des Fluids auf einen Wert T_{D} < T_{P}, der Verteilungswert genannt wird, jedoch höher ist als die Ausrottungstemperatur der Mikroorganismen,
- einen Kreis (16) zur Verteilung dieses Fluids auf der Temperatur T_{D} zu den Verteilungsstationen (46, 76) und
- einen Kreis (18) zum Wiedereinleiten ohne Stillstand des nicht verbrauchten Fluids, um es auf dieser Temperatur T_{D} zu halten, wobei der Wiedereinleitungskreis mit dem Verbindungs-T-Stück oder mit dem Mischer durch Venturi-Wirkung verbunden ist.

6. Sanitärbehandlungsvorrichtung nach Anspruch 5, **dadurch gekennzeichnet, dass** der Tauscher (30) ein Doppelstromtauscher ist, der die Temperatur des eintretenden Fluids um einen gegebenen Wert ΔT erhöht, um einen Wert T_{E} + ΔT zu erreichen, und die Temperatur T_{P} des pasteurisierten Fluids um denselben Wert verringert, um eine Verteilungstemperatur T_{D} = T_{P} - ΔT zu erreichen.

7. Sanitärbehandlungsvorrichtung nach Anspruch 5 oder 6, **dadurch gekennzeichnet, dass** der Pasteurisierungskreis (12) einen Primärkreis (14) mit einem Pasteurisierungstauscher (34) umfasst.

8. Sanitärbehandlungsvorrichtung nach einem der vorhergehenden Ansprüche 5, 6 oder 7, **dadurch gekennzeichnet, dass** der Pasteurisierungskreis (12) einen Pasteurisierungsbogen (40) umfasst.

9. Sanitärbehandlungsvorrichtung nach einem der Ansprüche 5 bis 8, **dadurch gekennzeichnet, dass** sie eine Heilableitung (72) oder eine thermische Nebenleitung umfasst, die zwischen den Pasteurisierungskreis (12) und den Verteilungskreis (16) eingefügt ist.

10. Sanitärbehandlungsvorrichtung nach einem der Ansprüche 5 bis 9, **dadurch gekennzeichnet, dass** sie einen einfachen Tauscher (58) umfasst, der in dem Wiedereinleitungskreis (18) angebracht ist.
